# EUROPÄISCHE PATENTSCHRIFT

(11) **EP 4 403 545 B1**
(45) Veröffentlichungstag und Bekanntmachung des Hinweises auf die Patenterteilung: **25.06.2025**
(21) Anmeldenummer: 23152173.3
(22) Anmeldetag: 18.01.2023
(51) Int. Cl.: C07C 45/50, C07C 47/02, C07F 9/6574

(54) **GEMISCH ZWEIER KONSTITUTIONSISOMEREN BISPHOSPHITE**
MIXTURE OF TWO CONSTITUENT ISOMERS OF BISPHOSPHITES
MÉLANGE DE DEUX ISOMÈRES DE CONSTITUTION À PHOSPHITES

(43) Veröffentlichungstag der Anmeldung: 24.07.2024
(73) Patentinhaber: Evonik Oxeno GmbH & Co. KG, 45772 Marl (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); KNOSSALLA, Johannes, 46514 Gahlen (DE); KUCMIERCZYK, Peter, 44628 Herne (DE); MARKOVIC, Ana, 45721 Haltern am See (DE)
(74) Vertreter: Evonik Patent Association

(56) Entgegenhaltungen:
- EP-A1- 4 074 720
- WO-A1-2014/056732

## Beschreibung

Die Erfindung betrifft ein Gemisch zweier konstitutionsisomeren Bisphosphite und deren Verwendung in der Hydroformylierung.

In EP 2 906 572 B1 wird ein Gemisch von konstitutionsisomeren Bisphosphiten (2a + 2b) beschrieben.

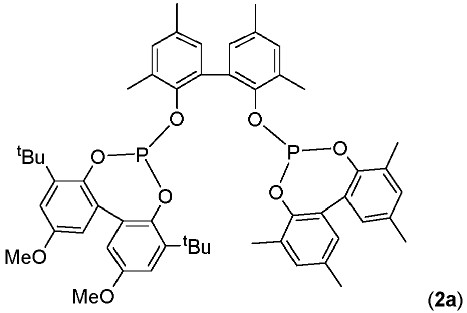

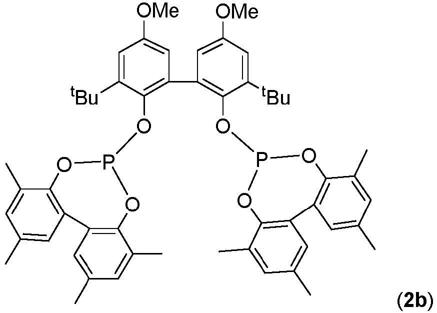

Die technische Aufgabe der Erfindung ist die Bereitstellung eines Gemisches, mit welchem bei der Hydroformylierung von Olefinen eine gesteigerte Ausbeute erzielt werden kann.

Die Aufgabe wird gelöst durch ein Gemisch gemäß Anspruch 1.

Gemisch umfassend die Verbindungen (1a) und (1b):

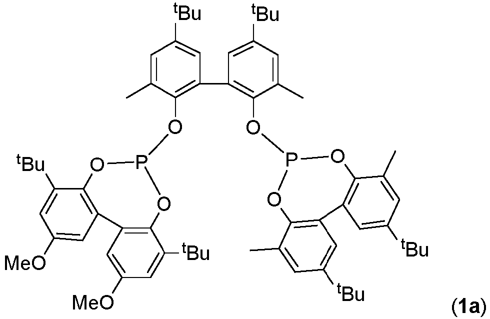

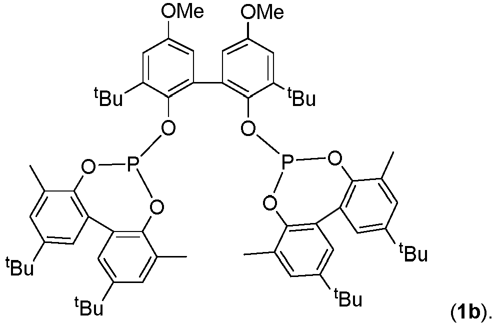

Neben dem Gemisch an sich wird auch ein Verfahren beansprucht, in welchem das Gemisch zum Einsatz kommt.

Verfahren umfassend die Verfahrensschritte:
a) Vorlegen eines Olefins;
b) Zugabe eines zuvor beschriebenen Gemisches;
c) Zugabe einer Rh-Verbindung;
d) Zuführen von H₂ und CO,
e) Erwärmen des Reaktionsgemisches aus a) bis d), wobei das Olefin zu einem Aldehyd umgesetzt wird.

In einer Variante des Verfahrens ist das Olefin in Verfahrensschritt a) ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Variante des Verfahrens ist die Rh-Verbindung ausgewählt aus: Rh(acac)(CO)₂, [(acac)Rh(COD)] (Umicore, acac = Acetylacetonat-Anion; COD = 1,5-Cyclooctadien), Rh₄CO₁₂.

In einer Variante des Verfahrens ist die Rh-Verbindung Rh(acac)(CO)₂.

In einer Variante des Verfahrens erfolgt das Zuführen von H₂ und CO in Verfahrensschritt d) mit einem Druck in dem Bereich von 1 bis 6 MPa (10 bis 60 bar).

In einer Variante des Verfahrens erfolgt das Erwärmen des Reaktionsgemisches in Verfahrensschritt e) auf eine Temperatur in dem Bereich von 80 °C bis 160 °C.

Im Folgenden soll die Erfindung anhand eines Ausführungsbeispieles näher erläutert werden.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das Substrat (1-Octen) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 100 ppm Rh(acac)(CO)₂ und die entsprechende Menge an Phosphitverbindung (L:Rh = 50:1) eingewogen und mit 8,0 ml Toluol aufgefüllt. Die jeweils eingebrachte Masse an Toluol wurde für die GC-Analyse bestimmt. Anschließend wurden 1,80 g 1-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 17 bar erhöht und die Reaktion bei konstantem Druck für 1 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m x 0,2 mm x 0,5 µm. Die quantitative Bestimmung von Restolefin und Aldehyd erfolgte gegen das Lösungsmittel Toluol als interner Standard.

Der Versuch wurde mit den Gemischen (**1a** + **1b**) und (**2a + 2b**) durchgeführt.

Das Gemisch (**2a + 2b**) dient hierbei als Vergleich.

### Ergebnisse der Katalyseversuche

[Rh]: 100 ppm, p: 17 bar, T: 120 °C; t: 1 h

**Tabelle: Hydroformylierung von 1-Octen**

| Ligand | Ausbeute [%] |
|---|---|
| (**1a + 1b**)* | 24 |
| (**2a + 2b**) | 16 |

| | |
|---|---|
| * erfindungsgemäßes Gemisch | |

Die durchgeführten Versuche belegen, dass die gestellte Aufgabe durch ein erfindungsgemäßes Gemisch gelöst wird.

## Patentansprüche

1. Gemisch umfassend die Verbindungen (**1a**) und (**1b**):

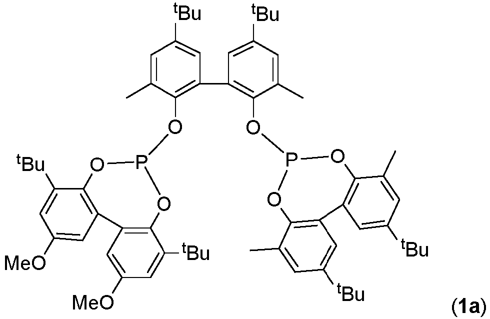

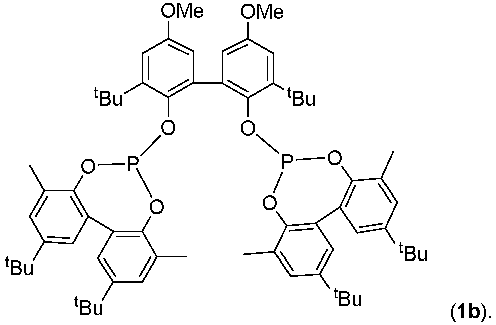

2. Verfahren umfassend die Verfahrensschritte:
a) Vorlegen eines Olefins;
b) Zugabe eines Gemisches nach Anspruch 1;
c) Zugabe einer Rh-Verbindung;
d) Zuführen von H₂ und CO,
e) Erwärmen des Reaktionsgemisches aus a) bis d), wobei das Olefin zu einem Aldehyd umgesetzt wird.

3. Verfahren nach Anspruch 2,
wobei das Olefin in Verfahrensschritt a) ausgewählt ist aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

4. Verfahren nach einem der Ansprüche 2 oder 3,
wobei die Rh-Verbindung ausgewählt ist aus: Rh(acac)(CO)₂, [(acac)Rh(COD)] (Umicore, acac = Acetylacetonat-Anion; COD = 1,5-Cyclooctadien), Rh₄CO₁₂.

5. Verfahren nach einem der Ansprüche 2 bis 4,
wobei die Rh-Verbindung Rh(acac)(CO)₂ ist.

6. Verfahren nach einem der Ansprüche 2 bis 5,
wobei das Zuführen von H₂ und CO in Verfahrensschritt d) mit einem Druck in dem Bereich von 1 bis 6 MPa (10 bis 60 bar) erfolgt.

7. Verfahren nach einem der Ansprüche 2 bis 6,
wobei das Erwärmen des Reaktionsgemisches in Verfahrensschritt e) auf eine Temperatur in dem Bereich von 80 °C bis 160 °C erfolgt.

## Claims

1. Mixture comprising the compounds (1a) and (1b):

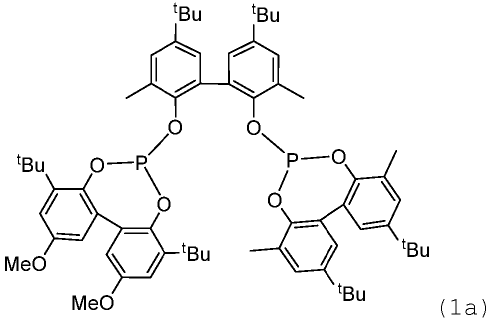

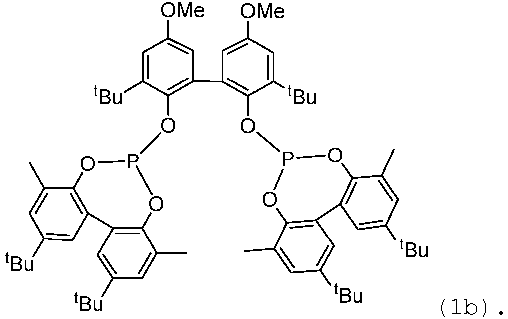

2. Process comprising the process steps of:
a) initially charging an olefin;
b) adding a mixture according to Claim 1;
c) adding a Rh compound;
d) feeding in H₂ and CO,
e) heating the reaction mixture from a) to d), to convert the olefin to an aldehyde.

3. Method according to Claim 2,
wherein the olefin in process step a) is selected from: ethene, propene, 1-butene, cis- and/or trans-2-butene, isobutene, 1,3-butadiene, 1-pentene, cis- and/or trans-2-pentene, 2-methyl-1-butene, 3-methyl-1-butene, 2-methyl-2-butene, hexene, tetramethylethylene, heptene, 1-octene, 2-octene, di-n-butene, or mixtures thereof.

4. Process according to either of Claims 2 and 3,
wherein the Rh compound is selected from: Rh(acac) (CO)₂, [(acac)Rh(COD)] (Umicore, acac = acetylacetonate anion; COD = 1,5-cyclooctadiene), Rh₄CO₁₂.

5. Method according to any of Claims 2 to 4,
wherein the Rh compound is Rh(acac) (CO)₂.

6. Method according to any of Claims 2 to 5,
wherein the H₂ and CO in process step d) is fed in at a pressure in the range of 1 to 6 MPa (10 to 60 bar).

7. Method according to any of Claims 2 to 6,
wherein the heating of the reaction mixture in process step e) is to a temperature in the range of 80°C to 160°C.

## Revendications

1. Mélange comprenant les composés (1a) et (1b) :

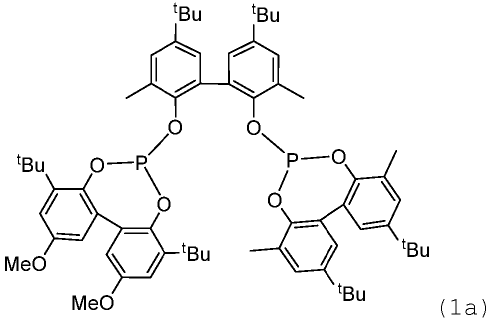

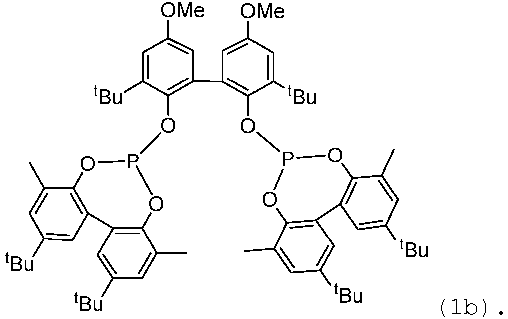

2. Procédé comprenant les étapes de procédé :
a) disposition préalable d'une oléfine ;
b) ajout d'un mélange selon la revendication 1 ;
c) ajout d'un composé de Rh ;
d) introduction de H₂ et de CO,
e) chauffage du mélange réactionnel provenant de a) à d), l'oléfine étant convertie en un aldéhyde.

3. Procédé selon la revendication 2,
l'oléfine dans l'étape de procédé a) étant choisie parmi : l'éthylène, le propène, le 1-butène, le cis-2-butène et/ou le trans-2-butène, l'isobutène, le 1,3-butadiène, le 1-pentène, le cis-2-pentène et/ou le trans-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

4. Procédé selon l'une des revendications 2 ou 3,
le composé de Rh étant choisi parmi : Rh(acac) (CO)₂, [(acac)Rh(COD)] (Umicore, acac = anion d'acétylacétonate ; COD = 1,5-cyclooctadiène), Rh₄CO₁₂.

5. Procédé selon l'une des revendications 2 à 4,
le composé de Rh étant Rh(acac) (CO)₂.

6. Procédé selon l'une des revendications 2 à 5,
l'introduction de H₂ et de CO dans l'étape de procédé d) ayant lieu à une pression dans la plage de 1 à 6 MPa (10 à 60 bars).

7. Procédé selon l'une des revendications 2 à 6,
le chauffage du mélange réactionnel dans l'étape de procédé e) ayant lieu à une température dans la plage de 80°C à 160°C.
